# EUROPEAN PATENT APPLICATION

(11) **EP 3 660 048 A1**
(43) Date of publication of application: **03.06.2020**
(21) Application number: 18306583.8
(22) Date of filing: 29.11.2018
(51) Int. Cl.: C07K 16/28, A61P 37/00

(54) **TREATMENT OF ACUTE GVHD USING DONOR-SPECIFIC ANTI-HLA ANTIBODIES**

(71) Applicant: ASSISTANCE PUBLIQUE - HÔPITAUX DE PARIS, 75004 Paris (FR)
(72) Inventor: ZUBER, Julien, 75020 Paris (FR); JOLLET, Isabelle, 86170 Cisse (FR); TAUPIN, Jean-Luc, 75004 Paris (FR)
(74) Representative: Regimbeau

(57) **Abstract**

The present invention is in the field of graft versus host disease treatment and relates to a composition for use in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor.

## Description

### TECHNICAL FIELD OF THE INVENTION

The present invention is in the field of graft versus host disease treatment. More precisely, it relates to a composition for use in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor.

### BACKGROUND ART

Acute Graft-versus-Host Disease (GVHD) seldom occurs after solid organ transplantation, resulting from the migration of host-reactive mature donor T cells from the graft to host target tissues. The risk of acute GVHD however depends on the amount of T cells in the donor and the degree of donor/recipient incompatibility. A primary or acquired immune deficiency in the recipient is an additional risk factor. In particular, acute graft-versus-host disease after solid organ transplantation is mainly described after intestinal transplantation in 5 to 10% of patients. The risk is increased in the case of a multivisceral transplant (intestine + liver + pancreas), especially if the spleen is included, and/or if the recipient is very immunocompromised (splenectomy, primary immune deficiency, young age). The risk of GVHD is major when transplanting non-identical HLA hematopoietic cells, occurring in this setting in 20 to 50% of cases.

Mortality associated to GVHD remains very high (30%), linked to a major infectious risk. Infections result from the breakdown of intestinal mucosal barriers and the strengthening of immunosuppressive treatment. The mortality is even greater than 55% in the case of cortico-resistant GVHD (about 30% of acute GVHD) and peaks at 100% if there is no response to the second line of treatment using antithymocyte globulin or inolimomab (Socié G et al., Blood 2017). Higher mortality, up to 80%, was also observed in immunocompromised recipients, mostly due to the absence of efficient treatment (Fischer RT et al., Pediatric Transplantation 2014).

For graft-versus-host diseases occurring after solid organ transplantation or after hematopoietic cells transplant, treatment is not standardized, and is often based on the increase in ongoing immunosuppressive treatments. To date, treatment has been based primarily on the administration of high doses of steroids, sometimes in combination with calcineurin inhibitors. A number of patients are corticosteroid-resistant or become corticosteroid dependent. In these high morbidity-mortality situations, several studies have been evaluating the benefit of ruxolitinib Jakavi® (JAK1 /2 inhibitor), inolimomab (a monoclonal antibody against CD25), or rabbit anti-thymocyte globulins (ATG). Neither early introduction of ATG nor the substitution of ATG by an investigational drug have dramatically improved the dismal outcome of these patients. Moreover, heightened immunosuppression increases the risk of lethal infections and may prevent the clearance of donor T cells by the host immune system. In contrast, immunosuppression tapering may allow host T cells to balance out their donor counterparts, but can, on the other hand, unleash a harmful two-way alloreactivity leading to either graft rejection or GVHD worsening (Perri R et al., Liver Transpl. 2007).

The bottom line is that none of these two conflicting strategies have yielded satisfactory results, and a new approach is critically warranted to improve patient survival (Perri R et al., Liver Transpl. 2007; Chen XB et al., World J Gastroenterol. 2012).

The presence of donor-specific anti-HLA antibodies (DSA, Donor Specific Antibody) in the recipient at the time of intestinal transplantation has been observed to be associated with a lower occurrence of graft-versus-host disease (Mazariegos GV et al., Am J Transplant 2004). Further studies, later showed that high-titer DSA could bind in-vivo to circulating donor cells and thus providing elimination of donor T lymphocytes in peripheral blood. (Zuber J et al., Am J Transplant 2015; Zuber J et al., Sci Immunol 2016). Thus, it was suggested that presence of DSA in the transplant recipient might serve as a biomarker for reduced risks of GVHD.

However, this hypothesized protective effect was only observed in the case where transplant recipients were continuously producing such DSAs before the transplantation. Similarly, the lessons drawn from hematopoietic stem cell transplantation told us that preformed DSA could be associated with primary graft failure (Morin-Zorman Front Immunol 2016), but were no longer a concern once donor engraftment had occurred. It was thus unclear whether post-transplant administration of a limited amount of DSA (at a target level low enough to avoid graft rejection), transiently detected in patient serum, would be sufficient to clear circulating donor T cells responsible of acute GVHD and thus treat acute GVHD without inducing graft rejection. Indeed, the presence of significant DSA titers before transplantation was also known to be associated to a higher risk of graft rejection (Patel R, Terasaki PI N Engl J Med. 1969).

### SUMMARY OF THE INVENTION

In the context of the present invention, the inventors unexpectedly discovered that the transient passive administration of a composition comprising DSA could efficiently treat transplantation recipients suffering from acute GVHD without inducing graft rejection.

Advantageously, the use of this composition allowed not only a very rapid improvement of life-threatening conditions, but also a decrease in immunosuppressive treatment, particularly corticosteroid therapy. Indeed, the inventors unexpectedly found that the transfer of donor-specific anti-HLA antibodies preferentially targeted the donor's activated T cells, thus preserving immune reconstitution from donor hematopoietic precursors. Moreover, the passive administration of donor-specific anti-HLA antibodies (such as a plasma) is a minimally invasive technic, with low iatrogenicity (particularly infectious) especially compared to the escalation of very potent immunosuppressive treatments (such as corticosteroids or ruxolitinib). Another significant advantage of the use of donor-specific anti-HLA antibodies is that a transient use is sufficient to permanently remove donor activated immune cells and treat acute GVHD, whereas immunosuppressive drugs limit their activation but do not get rid of them. The new therapy of acute GVHD proposed by the inventors is thus not only less invasive, but also transient, while other therapies of GVHD may require long-term treatment when GVHD becomes cortico-dependent. Furthermore, the plasma dose, needed to control GVHD, may be finely-tuned and highly tailored, based on the daily response and tolerance of treatment.

The administration of donor-specific anti-HLA antibodies to neutralize graft-versus-host reactivity is an innovative strategy that has never been reported, including in experimental models. Altogether, the inventors' findings show that the composition for use according to the invention provides an innovative alternative for the treatment of acute GVHD after transplantation of non-HLA-identical solid organs or hematopoietic cells.

The present invention thus relates to a composition for use in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor, wherein said composition comprises at least one donor-specific anti-HLA antibody. The composition is preferably a pharmaceutical composition, and may further comprise any pharmaceutically acceptable carrier.

The present invention also relates to the use of a composition comprising at least one donor-specific anti-HLA antibody in the manufacture of a medicament for use in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor.

The present invention also relates to the use of a composition comprising at least one donor-specific anti-HLA antibody in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor.

The present invention also relates to a method for treating acute Graft-versus-Host Disease (GVHD) in a transplantation recipient after allogenic transplantation with at least one solid organ and/or hematopoietic cells (HC) from a human leucocyte antigen (HLA)-mismatched transplantation donor, comprising administering to said transplantation recipient a therapeutically effective amount of a composition comprising at least one donor-specific anti-HLA antibody.

Preferably, said composition comprises plasma or serum from a sensitized plasma-donor or blood-donor producing antibodies specifically binding to at least one donor-specific HLA-antigen, preferably said sensitized plasma-donor or blood-donor is a healthy female plasma-donor or blood-donor, whose blood, plasma or serum has previously been screened for anti-HLA antibodies following pregnancy.

In the context of the invention, said composition may notably have been obtained beforehand by:
a) interrogating databases including healthy female plasma or blood donors, whose blood, plasma or serum had been screened for anti-HLA antibodies following pregnancy, and selecting at least one healthy female plasma-donor or blood-donor likely to produce a donor-specific anti-HLA antibody
b) testing plasma or serum sample(s) from the selected healthy female plasma-donor(s) or blood-donor(s) for anti-HLA antibodies and selecting the plasma or serum sample if said plasma or serum sample comprises donor-specific anti-HLA antibodies, and
c) optionally, diluting the plasma or serum sample.

Alternatively, said composition may comprise one or more monoclonal donor-specific anti-HLA antibody(ies).

In one embodiment, said acute GVHD occurs after allogenic transplantation with at least one solid organ from an HLA-mismatched transplantation donor, and said at least one solid organ is preferably selected from kidney, small bowel, liver, pancreas, spleen, lung and their combinations. In this case, the at least one donor-specific anti-HLA antibody may be a donor-specific anti-HLA class I or a donor-specific anti-HLA class II antibody.

In another embodiment, said acute GVHD occurs after allogenic transplantation with hematopoietic cells from an HLA-mismatched transplantation donor, preferably after allogenic bone marrow transplantation or allogenic hematopoietic stem cell (HSC) transplantation from an HLA-mismatched transplantation donor, or after blood transfusion from an HLA-mismatched transplantation donor (since blood transfusion is another case of hematopoietic cells transplantation). In this case, the at least one donor-specific anti-HLA antibody is preferably a donor-specific anti-HLA class II antibody. Preferably, said composition is for use when said acute GVHD is a corticosteroid-resistant GVHD or a corticosteroid-dependent GVHD.

### DESCRIPTION OF THE FIGURES

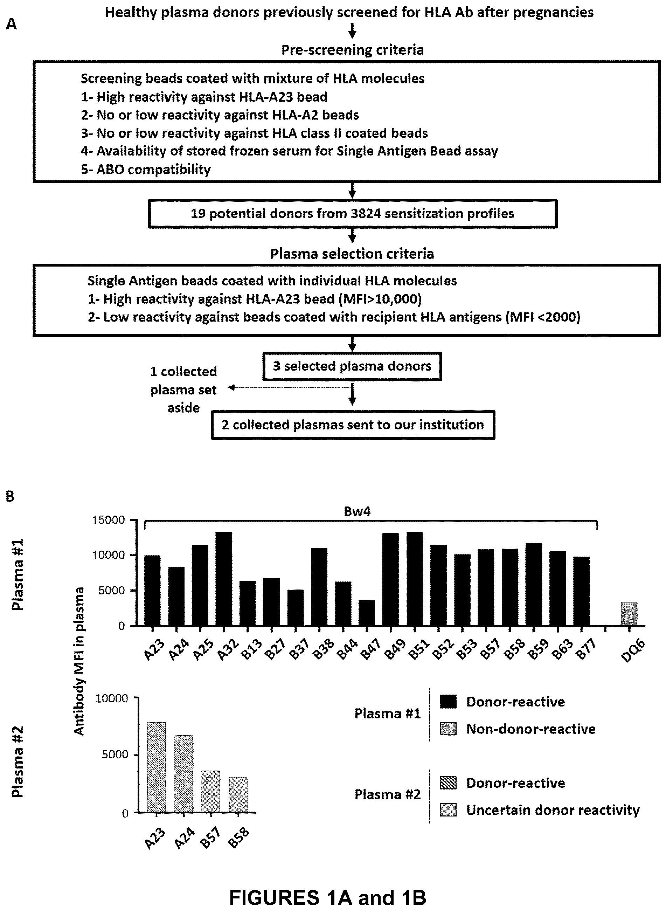

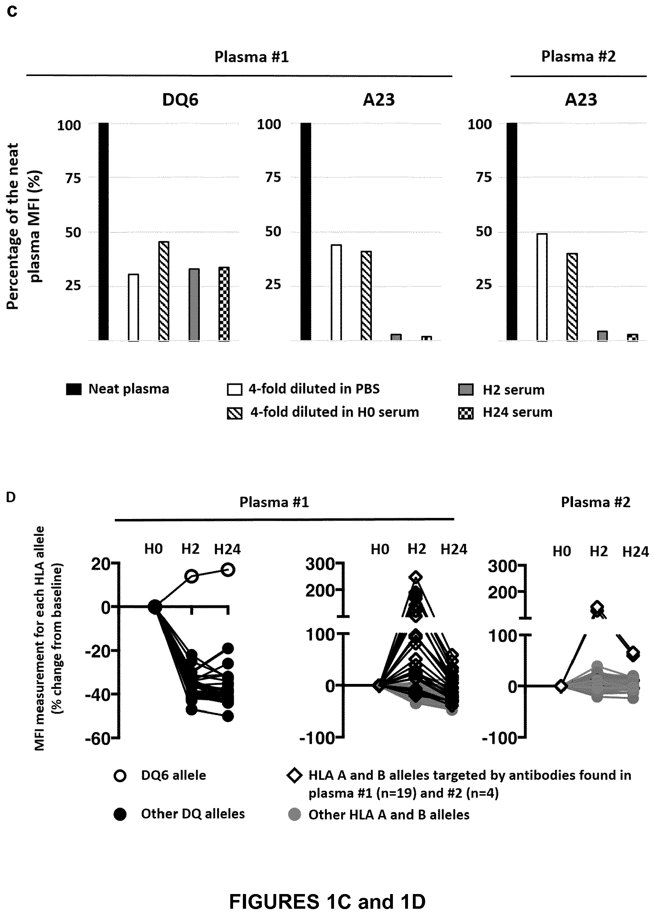

***FIGURE 1******: PLASMA SELECTION, CHARACTERIZATION AND ADMINISTRATION***
   A) Flowchart showing the selection process of DSA-rich plasmas through a nationwide screening. Thirteen HLA laboratories located in 16 EFS centers interrogated databases including healthy plasma female donors, whose serum had been screened for anti-HLA antibody following pregnancy. The prescreening step aimed at identifying plasmas from registries that exhibited a strong reactivity against the unique screening bead coated with HLA-A23 but not HLA-A2. Seven centers identified 16 putative donors fulfilling plasma preselection criteria, 5 of which were eventually selected after Single Antigen (SA) Luminex assay had confirmed the adequate sensitization profile on historical sera. The 5 putative donors were informed and invited for plasma donation. Plasmas were collected, frozen and stored according to the EFS standard process. Two donors were eventually discarded because of changes in the anti-HLA sensitization profile in the serum collected at the time of plasma donation. Two frozen plasmas were sent to our institution; the third one was set aside at the EFS. B) In order to ensure MFI comparability, anti-HLA antibody levels in selected plasmas were eventually assessed at the Immunology Laboratory in Saint-Louis Hospital, where patient's circulating anti-HLA antibodies were monitored over time. Only the most significant anti-HLA antibodies, with MFI greater than 3,000, are depicted on this graph. The black bracket indicates HLA antigens sharing the public Bw4 epitope targeted by the DSA in plasma #1. It was unclear whether the second plasma contained one antibody targeting a single epitope shared by a few Bw4-associated HLA molecules, including A23, or several distinct antibodies. C) Anti-HLA antibody level as a percentage of the neat plasma level in ex-vivo 4-fold diluted plasma and in patient's serum. D) Antibody titer trajectory after plasma infusion, as percentage change from baseline.
      Abbreviations: Abs, antibodies; H0: right before the onset of plasma infusion; EFS stands for French National Blood Service; H2 and H24: two and twenty-four hours after the end of plasma administration, respectively; MFI, mean fluorescence intensity; PBS, phosphate buffered saline.

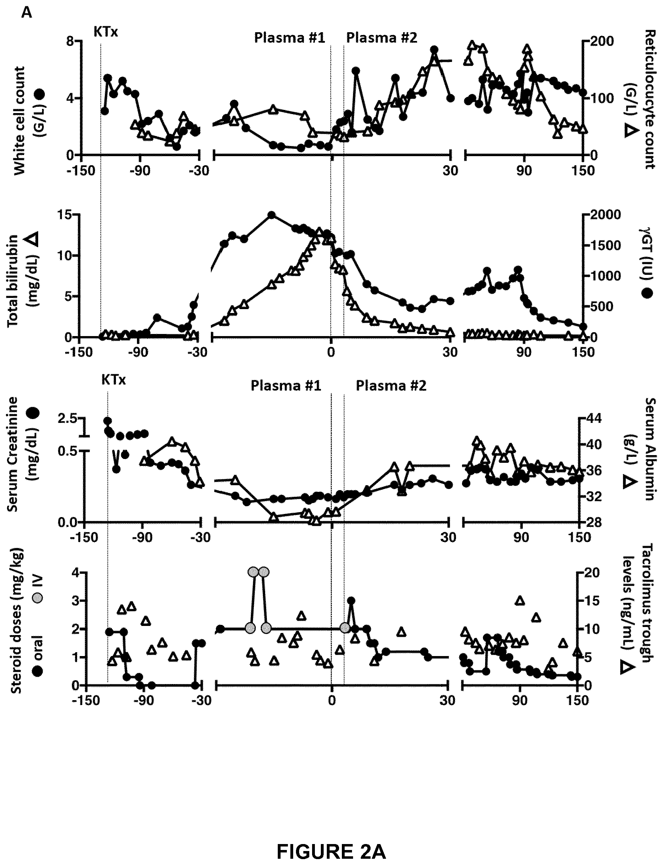

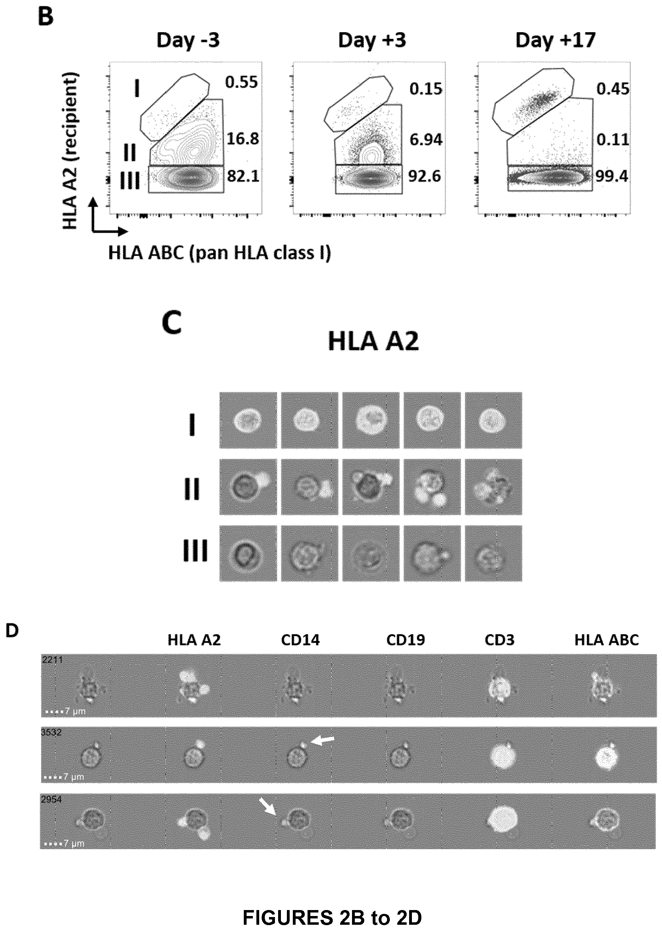

*FIGURE 2**: CLINICAL AND IMMUNOLOGICAL RESPONSES TO DSA-RICH PLASMA **ADMINISTRATION***
   A) White cell count, reticulocyte count, total bilirubin, yGT, plasma creatinine and serum albumin levels over time, and concurrent immunosuppressive regimen. Plasma #1 and Plasma #2 indicate the times of DSA-rich plasma administration. KTx indicates the day of kidney transplantation. B) Flow cytometry contour-plot gated on CD3+ T cells, plotting recipient-specific HLA allele (HLA A2) versus a pan-HLA class I staining. The upper gate (I) includes the recipient cells, characterized by proportional expression of recipient-specific HLA and pan HLA class I. The lower gate (III) includes the donor cells, which expressed pan HLA class I but not recipient-specific HLA antigen. The intermediate gate (II) includes cells that expressed mild levels of recipient-specific HLA antigen. C) Imaging of circulating CD3+ cells using Amnis® ImageStream showed that recipient microvesicles, bound to some donor T cells, accounted for the mild expression of recipient HLA antigen by cells in the subset II. D) Multicolor imaging (in gray scale), using a panel that combined CD3, CD14, CD19, HLA ABC, and HLA A2, identified a few recipient-derived vesicles that co-stained with the hallmark monocyte/macrophage marker CD14 (white arrows), but most of them remained of unknown origin.
      Abbreviations: Aza, azathioprine; IV, intra-venous; RBCP, red blood cell pack; Tac, tacrolimus.

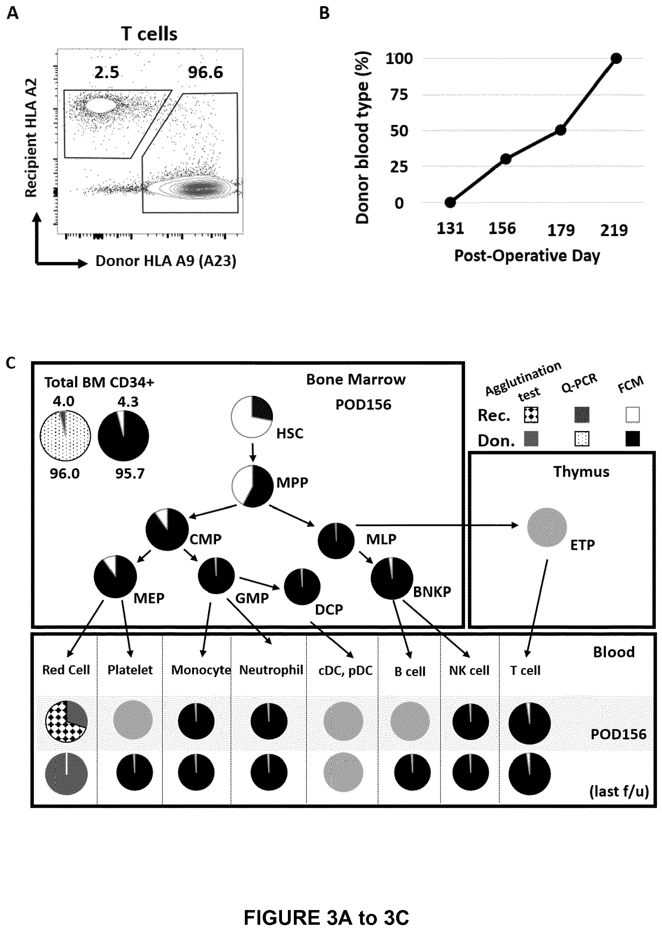

***FIGURE 3******: SUSTAINED AND HIGH LEVELS OF MULTILINEAGE DONOR CHIMERISM IN BLOOD AND BONE MARROW.***
   A) Representative flow-cytometry contour plot showing high level of donor T cell chimerism persisting after GVHD remission (POD179). B) Graduate conversion from 100% recipient (0 Rh D+ C+ E- c+ e+ K-) to 100% donor (0 Rh D+ C- E- c+ e+ K-) blood type. C) Assessment of donor chimerism in blood cell population and bone-marrow lineage-negative CD34+ progenitors through agglutination test, molecular biology and flow cytometry. Blood cell subsets were defined as follows: platelets (CD41a+), monocytes (CD11b+ CD14+ CD15- CD3-), neutrophils (CD11b+ CD14- CD15+ CD3-), B cells (CD19+ CD3-), NK cells (CD56+ CD3-) and T cells (CD3+). Hierarchical organization of the hematopoiesis is represented by a handful of lineage-negative CD34+ progenitors along a branched differentiation pathway: CMP (CD38+ CD10- CD123dim CD45RA-), DCP (CD38+ CD10-CD45RA+ CD23+), HSC (CD38- CD90+ CD45RA-), MPP (CD38- CD90- CD45RA-), MLP (CD38-CD45RA+); BNKP (CD38+ CD10+); GMP (CD38+ CD10-, CD45RA+ CD123+); MEP (CD38+ CD10-CD45RA- CD123-). Donor chimerism is not indicated (gray circles) whenever chimerism was not assessed.
      Abbreviations: BNKP, B and NK progenitors; BM, Bone Marrow; cDC, conventional dendritic cells; CDP, dendritic cell progenitors; CMP, common myeloid progenitor; ETP, early thymic progenitors; f/u, follow-up; GMP, Granulocyte-Monocyte Progenitors; HSC, Hematopoietic Stem Cell; MEP, Megakaryocyte Erythroid Progenitor; MLP, Multipotent Lymphoid Progenitor; MPP, Multi-Potent Progenitor; NK cell, Natural Killer cell; POD, pDC, plasmacytoid dendritic cells; Post-Operative Day; Q-PCR, quantitative polymerase chain reaction.

### DETAILED DESCRIPTION OF THE INVENTION

In the context of the present invention, the inventors unexpectedly discovered that the transient passive administration of a composition comprising DSA could efficiently treat transplantation recipients suffering from acute GVHD, without inducing graft rejection. Advantageously, the use of this composition allowed not only a very rapid improvement of life-threatening conditions, but also a decrease in immunosuppressive treatment, particularly corticosteroid therapy. Indeed, the inventors unexpectedly found that the transfer of donor-specific anti-HLA antibodies preferentially targeted the donor's activated T cells, thus preserving immune reconstitution from donor hematopoietic precursors. Moreover, the passive administration of donor-specific anti-HLA antibodies (such as a plasma) is a minimally invasive technic, with low iatrogenicity (particularly infectious) especially compared to the escalation of very potent immunosuppressive treatments (such as corticosteroids or ruxolitinib). Another significant advantage of the use of donor-specific anti-HLA antibodies is that a transient use is sufficient to permanently remove donor activated immune cells and treat acute GVHD, whereas immunosuppressive drugs limit their activation but do not get rid of them. The new therapy of acute GVHD proposed by the inventors is thus not only less invasive, but also transient, while other therapies of GVHD may require long-term treatment when GVHD becomes cortico-dependent. Furthermore, the plasma dose, needed to control GVHD, may be finely-tuned and highly tailored, based on the daily response and tolerance of treatment.

The administration of donor-specific anti-HLA antibodies to neutralize graft-versus-host reactivity is an innovative strategy that has never been reported, including in experimental models. Altogether, the inventors' findings show that the composition for use according to the invention provides an innovative alternative for the treatment of acute GVHD after transplantation of non-HLA-identical solid organs or hematopoietic cells.

### COMPOSITION FOR USE IN THE TREATMENT OF ACUTE GVHD

The invention concerns a composition for use in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor, wherein said composition comprises at least one donor-specific anti-HLA antibody. The composition is preferably a pharmaceutical composition, and may further comprise any pharmaceutically acceptable carrier.

The present invention also relates to the use of a composition comprising at least one donor-specific anti-HLA antibody in the manufacture of a medicament for use in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor.

The present invention also relates to the use of a composition comprising at least one donor-specific anti-HLA antibody in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor.

The present invention also relates to a method for treating acute Graft-versus-Host Disease (GVHD) in a transplantation recipient after allogenic transplantation with at least one solid organ and/or hematopoietic cells (HC) from a human leucocyte antigen (HLA)-mismatched transplantation donor, comprising administering to said transplantation recipient a therapeutically effective amount of a composition comprising at least one donor-specific anti-HLA antibody.

"Graft-versus-Host Disease" or "GVHD" is a complication occurring after allogenic transplantation, wherein donor immunocompetent lymphoid cells damage recipient tissues. For acute GVHD to occur, three criteria are prevalent (1) an immunological incompatibility between transplantation donor and the transplantation recipient, (2) immune cells present in the graft should be able to recognize the recipient and (3) an inability of the recipient to remove the donor's cells. Criterium (3) varies depending on the clinical setting, in particular on the equilibrium between the numbers of donor immunocompetent lymphoid cells and recipient immunocompetent lymphoid cells. For instance, solid organ transplantation generally results in the transplantation of a reduced number of donor immunocompetent lymphoid cells (except in cases of multivisceral transplantation, especially when the spleen is included), explaining a relatively low occurrence of acute GVHD in this setting, since recipient immunocompetent lymphoid cells are normally able to remove the donor's cells in this context. The situation is clearly different in the case of hematopoietic cells transplantation, due to former irradiation of the recipient and transplantation of multiple donor immunocompetent lymphoid cells, explaining the much higher occurrence of acute GVHD in this setting. The immune status of the recipient may also affect the occurrence of acute GVHD, since a particularly immunocompromised recipient may not be able to remove even a moderate number of donor immunocompetent lymphoid cells. Acute GVHD usually begins 10 to 30 days post transplantation as a mild or faint rash on the patient's back or abdomen; it can also appear on the hands or feet. The rash may spread and eventually develop into peeling or blistering that resemble toxic epidermal necrolysis. Fever may be present as well as watery or bloody diarrhea with stomach cramps indicating gastrointestinal involvement, jaundice (yellowing of the skin and eyes) and abnormal liver function tests indicating liver involvement. Also, transfusion-associated and solid organ transplantation-associated acute GVHD, unlike after Bone Marrow Transplantation (BMT), frequently results in pancytopenia secondary to marrow aplasia, with an ensuing mortality greater than 90%. On very rare occasions, GVHD can occur after blood transfusion. The risk of GVHD after transfusion is higher in immunocompromised recipients whose immune system is unable to recognize the transfused T-cells as "non-self".

By "treatment" is meant an improvement, observed at the clinical or biochemical level, of the patient's disease. In the context of acute GVHD, an improvement in any of rash, peeling or blistering, fever, watery or bloody diarrhea with stomach cramps, jaundice, abnormal liver function tests, and pancytopenia secondary to marrow aplasia will be considered as a treatment.

By "HLA", "HLA antigen" or 'HLA molecule" is meant human leukocyte antigens, which correspond to the antigens of the major histocompatibility complex (MHC) in humans. Present in all jawed mammals, MHC molecules are cell surface proteins, that allow "self" and "non-self" identification by cells of the immune system. Herein, "HLA" and "MHC" are equivalent and can be used interchangeably. Similarly, in the context of HLA or MHC, "molecule" and "antigen" are considered equivalent and can be used interchangeably. There are several types of HLA molecules, such as HLA class I molecules and HLA class II molecules.

With exception of red blood cells, all cells of jawed mammals carry HLA class I molecules on their surface. HLA class I molecules allow cells to be recognized as belonging to an organism ("self" identification). Additionally, HLA class I molecules, can trigger an immediate immune response against a particular antigen, by displaying peptide fragments of "non-self" proteins present within the cell. Such peptides, generally 8 to 10 amino acid long, are generated through degradation of cytosolic proteins by the proteasome, for example in the event of an infection.

HLA class I molecules are heterodimers consisting of two polypeptide chains, α (α1, α2 and α3) and β2-microglobulin. The two chains are linked noncovalently via interaction of b2m and the α3 domain. The α chain is polymorphic and encoded by an HLA gene. The α1 and α2 domains fold and make up a groove where displayed peptides bind, while α3 domain is responsible for interactions with T lymphocytes. Together, they allow the recognition of the coupled peptide for antigenicity by T lymphocytes.

HLA class II molecules are present on the surface of antigen-presenting cells such as monocytes, macrophages and activated lymphocytes (B or T cells).

As opposed to HLA class I antigens, antigens presented by class II molecules are derived from extracellular proteins. Loading of an HLA class II molecule occurs by phagocytosis; extracellular proteins are endocytosed, digested in lysosomes, and the resulting antigenic peptide fragments, generally 15 and 24 amino acid long, are loaded onto HLA class II molecules prior to their migration to the cell surface.

HLA class II molecules are also heterodimers, consisting of two polypeptide chains α (α1 and α2) and β (β1, β2). The α1 and β1 regions of the chains come together and make up a groove where the antigenic peptide binds.

This system is important for the initiation of immune response.

By "donor-specific" HLA antigen, it is meant that the HLA antigen is expressed by the HLA-mismatched transplantation donor but not expressed by the transplantation recipient.

By "donor specific anti-HLA antibody" or "donor specific antibody" or "DSA" is meant an antibody that specifically binds to at least one donor-specific HLA-antigen. Herein, "donor specific anti-HLA antibody", "donor specific antibody" and "DSA" are equivalent and can be used interchangeably.

By "antibody" or "immunoglobulin" is meant a molecule comprising at least one binding domain for a given antigen and a constant domain comprising an Fc fragment capable of binding to Fc receptors (FcR). In most mammals, like humans and mice, an antibody consists of four polypeptide chains: two heavy chains and two light chains bound together by a variable number of disulfide bridges providing flexibility to the molecule. Each light chain consists of a constant domain (CL) and a variable domain (VL); the heavy chains consisting of a variable domain (VH) and three or four constant domains (CH1 to CH3 or CH1 to CH4) according to the isotype of the antibody. In a few rare mammals, such as camels and llamas, the antibodies consist of only two heavy chains, each heavy chain comprising a variable domain (VH) and a constant region.

The variable domains are involved in epitope/antigen recognition, while the constant domains are involved in the biological, pharmacokinetic and effector properties of the antibody.

The variable region differs from one antibody to another. Indeed, the genes encoding antibody heavy and light chains are respectively generated by recombination of three and two distinct gene segments called VH, DH and JH-CH for the heavy chain and VL and JL-CL for the light chain. The CH and CL segments do not participate in recombination and form the constant regions of the heavy and light chains, respectively. Recombinations of the VH-DH-JH and VL-JL segments form the variable regions of the heavy and light chains, respectively.

By "epitope" is meant a site on an antigen recognized by an antibody. A given antigen may have may have several identical or different epitopes. T-epitopes are short peptides, derived from an antigen, presented on the surface of cells in order to be recognized by the immune system. B-epitopes are representative of the identity of an antigen in its entirety. The structure involved in recognition may be the primary structure, in this case the epitope relates to the amino acid sequence. Alternatively, the structure involved in recognition may be the tertiary structure, in such case the epitope relates to the tridimensional structure of the protein after folding.

Antibodies specifically recognizing a given antigen may be monoclonal or polyclonal. By "monoclonal antibody" or "monoclonal antibody composition" is meant a composition comprising antibody molecules having an identical and unique epitope specificity for a given antigen. The antibody molecules present in the composition may vary as regards their post-translational modifications, and notably as regards their glycosylation structures or their isoelectric point, but have all been produced by the same B lymphocyte clone and thus encoded by the same heavy and light chain sequences and therefore have, before any post-translational modification, the same protein sequence. Certain differences in protein sequences, related to post-translational modifications (such as for example the cleavage of the C-terminal lysine of the heavy chain, deamidation of asparagine residues and/or isomerization of aspartate residues), may nevertheless exist between the various antibody molecules present in the composition.

The antibody molecules present in the monoclonal antibody composition are likely to vary in terms of their post-translational modifications, and notably in terms of their glycosylation structures or their isoelectric point, but have all been encoded by the same heavy and light chain sequences and thus have, before any post-translational modification, the same protein sequence. Certain differences in protein sequences, related to post-translational modifications (such as for example cleavage of the heavy chain C-terminal lysine, deamidation of asparagine residues and/or isomerization of aspartate residues), may nevertheless exist between the various antibody molecules present in these compositions.

By "polyclonal antibody" or "polyclonal antibody composition" is meant a composition comprising a mix of antibody molecules produced by multiple B lymphocyte clones, generally having different epitope specificities towards a given unique antigen.

Antibodies specifically recognizing a given antigen may alternatively or further be chimeric, humanized or fully human.

By "chimeric" antibody is meant an antibody that contains a natural variable region (light chain and heavy chain) derived from an antibody of a given species in combination with the light and heavy chain constant regions of an antibody of a species heterologous to said given species. Advantageously, if the monoclonal antibody composition for use as a medicinal product according to the invention comprises a chimeric monoclonal antibody, the latter comprises human constant regions. From a non-human antibody, a chimeric antibody can be prepared by using the genetic recombination techniques well-known to a person skilled in the art. For example, the chimeric antibody can be prepared by cloning the heavy and light chains of a recombinant DNA comprising a promoter and a sequence encoding the variable region of the non-human antibody, and a sequence encoding the constant region of a human antibody. For methods for preparing chimeric antibodies, reference may be made, for example, to the document by Verhoeyen et al. BioEssays, 8:74, 1988 and Verhoeyen et al. Science, 239:1534-1536, 1988.

By "humanized" antibody is meant an antibody that contains CDRs derived from an antibody of non-human origin, the other portions of the antibody molecule being derived from one (or from several) human antibodies. Moreover, certain residues of the framework regions (FR) may be modified to retain binding affinity (Jones *et al.*-1986; Verhoeyen *et al.* 1988; Riechmann *et al.*-1988). The humanized antibodies according to the invention can be prepared by techniques known to a person skilled in the art such as CDR grafting, resurfacing, superhumanization, human string content, FR libraries, guided selection, FR shuffling and humaneering technologies, as summarized in the review by Almagro et al. Frontiers in Bioscience 13, 1619-1633, January 1, 2008.
by "human" antibody, it is meant an antibody whose entire sequence is of human origin, that is to say whose coding sequences have been produced by recombination of human genes encoding antibody heavy and light chains. Indeed, it is now possible to produce transgenic animals (eg mice) that are capable, upon immunization, of producing a complete repertoire of human antibodies in the absence of endogenous immunoglobulin production (see Jakobovits et al. -1993 (a) and (b), Bruggermann et al., 1993, Duchosal et al., 1992, US patents 5,591,669, 5,598,369, 5,545,806, 5,545,807, 6,150,584). Human antibodies can also be obtained from phage display libraries (Hoogenboom et al 1991, Marks et al 1991, Vaughan et al 1996).

### ORIGIN OF THE ANTIBODY(IES) SPECIFICALLY BINDING TO AT LEAST ONE DONOR-SPECIFIC HLA-ANTIGEN

The at least one antibody specifically binding to at least one donor-specific HLA-antigen comprised in the composition may have various origins, as described below.

### Compositions derived from blood and/or plasma

Preferably, said composition comprises plasma or serum from a sensitized plasma-donor or blood-donor producing donor-specific anti-HLA antibodies, preferably said sensitized plasma-donor or blood-donor is a healthy female plasma-donor or blood-donor, whose blood, plasma or serum has previously been screened for anti-HLA antibodies following pregnancy.

It may be advantageous to use compositions derived from plasma or serum from a sensitized plasma-donor or blood-donor since these fluids naturally comprise antibodies, particularly anti-HLA antibodies, from said plasma or blood-donor.

In addition, many countries, facilities collecting blood and/or plasma donations hold databases gathering characteristics of blood, plasma or serum, including the presence of antibodies specifically binding to HLA antigens. Antibodies to human leukocyte antigens (HLA) in donated plasma have been implicated as a cause of transfusion-related acute lung injury (TRALI). A potential measure to reduce the risk of TRALI includes screening of plasma donors for HLA-targeted antibodies (Triulzi DJ Transfusion 2009). The prevalence of HLA antibodies increases significantly with more pregnancies (1.7% (0), 11.2% (1), 22.5% (2), 27.5% (3), 32.2% (4 or more pregnancies)). In France, a database is hosted by the EFS (Etablissement Francais du Sang), and indexes information on anti-HLA antibodies comprised in collected blood, plasma and/or serum. These databases are designed to deliver specific information to health professionals and therefore are easily accessible to clinicians.

Advantageously, said blood, plasma and/or serum can be collected from healthy women following pregnancy. Indeed, 30% of multiparous pregnant women develop anti-HLA antibodies against HLA-antigens of paternal origin expressed by the fetus. Even though they may disappear quickly after pregnancy, they sometimes persist for years.

Blood and/or plasma donations, especially when collected from healthy women following pregnancy, thus allow the collection and indexing of a large variety of different antibodies, particularly anti-HLA antibodies, at low expense.

### Compositions derived from plasma

In a first embodiment, said composition comprises plasma from a sensitized plasma-donor or blood-donor producing donor-specific anti-HLA antibodies.

By "plasma" or "blood plasma" is meant is a blood fraction cleared of cells. Plasma is the supernatant fraction obtained after centrifugation of the blood in the presence of coagulation inhibitor. Therefore, plasma contains all the proteins present in the blood including antibodies, clotting proteins (coagulation factors and fibrinogen) and others. In many countries, a plasma donation system is implemented. Plasma collection is realized through a process called plasmapheresis, wherein blood is collected in a vein, and using a blood separating machine, plasma is separated from other blood components by centrifugation and then collected in a pouch. Other blood components such as leukocytes, erythrocytes and thrombocytes are returned to the blood circulation of the donor.

### Compositions derived from serum

In another embodiment, said composition comprises a serum from a sensitized plasma-donor or blood-donor producing donor-specific anti-HLA antibodies.

By "serum" or "blood serum" is meant is a blood fraction cleared of cells and clotting proteins. Serum is the supernatant fraction obtained after coagulation and centrifugation of the blood in the absence of coagulation inhibitor. Therefore, blood serum is a blood plasma devoid of clotting proteins (coagulation factors and fibrinogen).

Preferably said sensitized plasma-donor or blood-donor is a healthy female plasma-donor or blood-donor, whose blood, plasma or serum has previously been screened for the presence anti-HLA antibodies following pregnancy.

### Blood group of the donor

Preferably, said sensitized plasma-donor or blood-donor has a compatible blood group with respect to the transplantation recipient.

By "blood group" is meant the blood classification, based on the presence and absence of inherited antigenic substances on the surface of red blood cells. The two most significant group systems for analyzing human blood compatibilities are the ABO blood group system and Rh blood group system. The ABO system relies on the presence or absence of the A and/or B alleles of the ABO gene located on chromosome 9. Thus, human beings may be classified into four groups in regard of their antigen properties, those with antigen A (group A), those with antigen B (group B), those with both antigen A and B (group AB) and those with no antigen (group O). In the Rh (Rhesus) system, the most significant Rh antigen is the D antigen, because it is the most likely to provoke an immune system response of the five main Rh antigens. The presence or absence of the Rh(D) antigen is signified by the + or - sign. For example, a O⁽⁻⁾ person is considered as a universal donor as it does not display any antigen at the surface of its red blood cells and will thus not be recognized by antibodies and lymphocytes directed against blood group antigens that might be present in the donor; whereas an AB⁽⁺⁾ person is considered to be a universal receiver as he is not immunoreactive against ABO and Rh antigens.

In a preferred embodiment, said sensitized plasma-donor or blood-donor is of the O⁽⁻⁾ blood group, since it is then blood compatible with any recipient.

In another preferred embodiment, said sensitized plasma-donor or blood-donor has the same blood group as the transplantation recipient.

### Obtention of said composition derived from plasma and/or serum

In the present invention, said composition may preferably have been obtained beforehand by:
a) interrogating databases including healthy female plasma or blood donors, whose blood, plasma or serum had been screened for anti-HLA antibodies following pregnancy, and selecting at least one healthy female plasma-donor or blood-donor likely to produce a donor-specific anti-HLA antibody,
b) testing plasma or serum sample(s) from the selected healthy female plasma-donor(s) or blood-donor(s) for anti-HLA antibodies and selecting the plasma or serum sample if said plasma or serum sample comprises donor-specific anti-HLA antibodies, and
c) optionally, diluting the plasma or serum sample.

In first step a), databases are interrogated to identify donors whose blood, plasma or serum has been screened for anti-HLA antibodies.

Transfusion-related acute lung injury (TRALI) is a major cause of transfusion-related mortality and morbidity. It is associated with the presence of anti-leukocyte antibodies in donor plasma.

For this reason, all blood and plasma donations are screened for the presence of anti-HLA antibodies. However, for cost reasons, a Multiple Antigens assay rather than a Single Antigen assay is generally used.

A "Single Antigen assay" is herein intended to mean a solid phase assay in which:
i1) the sample to be analyzed is incubated with a mixture of microbeads distinguishable in flow cytometry analysis, each bead being coated with only one single HLA antigen;
ii1) the resulting sample is further incubated with a secondary antibody, conjugated with the R-phycoerythrin (PE) fluorophore reporter; and
iii1) the resulting sample is analyzed by flow cytometry and PE mean fluorescent intensity is measured.

In step ii1) above, the secondary antibody is an antibody that is able to bind to all anti-HLA antibodies that may be present in the plasma or serum sample to be analyzed, such as an antibody directed to the constant region of anti-HLA antibodies that may be present in the plasma or serum sample to be analyzed. For instance, an antibody directed to human IgG constant region may be used.

Washing steps may be included between steps i1) and ii1), and/or between steps ii1) and iii1).

Luminex® Single Antigen assay is one such assay. This assay comprises solubilized HLA molecules immobilized on polystyrene microbeads impregnated with a unique mixture of two fluorescent dyes that are simultaneously excited by a red laser at 635 nm. The emitted light is then detected at wavelengths of 660 nm (red) and 730 nm (infrared) using a flow cytometer. By measuring the emission intensities for both channels, up to 100 distinct beads with a unique HLA antigen can be identified concomitantly. The detection of HLA antibodies is achieved by using a secondary antibody conjugated with the R-phycoerythrin (PE) fluorophore reporter which is excited by a green laser (532 nm) and detected at 576 nm. These beads carry only one or specific allele. Such assay permits qualitative and quantitative determination of anti-HLA antibodies against a given HLA antigen and may notably be performed using Single Antigen Luminex kit from One Lambda® "LABScreen® Single Antigen".

A "Multiple Antigens assay" is herein intended to mean a solid phase assay in which:
i2) the sample to be analyzed is incubated with a mixture of microbeads distinguishable in flow cytometry analysis, each bead being coated with multiple distinct HLA antigens;
ii2) the resulting sample is further incubated with a secondary antibody, conjugated with the R-phycoerythrin (PE) fluorophore reporter; and
iii2) the resulting sample is analyzed by flow cytometry and PE mean fluorescent intensity is measured.

In step ii2) above, the secondary antibody is an antibody that is able to bind to all anti-HLA antibodies that may be present in the plasma or serum sample to be analyzed, such as an antibody directed to the constant region of anti-HLA antibodies that may be present in the plasma or serum sample to be analyzed. For instance, an antibody directed to human IgG constant region may be used.

Washing steps may be included between steps i2) and ii2), and/or between steps ii2) and iii2).

Such Multiple Antigens assay is generally performed using kits sold for detecting the presence of anti-HLA class I or anti-HLA class II antibodies, which comprise beads bearing multiple HLA antigens, rather than beads coated with a single HLA antigen. An example of such kits is the Luminex kit from One Lambda® "LABScreen® PRA".

Nevertheless, kits sold for detection of anti-HLA class I or anti-HLA class II antibodies however in general include multiple types of beads, and a skilled person will know which HLA molecules are bound to each screening bead. As an example, the Labscreen® PRA Class I kit includes 57 different types of beads (numbered from 1-36, 69-89, 95 and 97), all grafted with a maximum of 6 HLA class I antigens. Among these beads, 15 beads (No 10, 11, 12, 13, 18, 19, 20, 22, 24, 25, 26, 36, 73, 75, 81) are grafted with an HLA A2 antigen. Consequently, if all the beads bearing an HLA A2 molecule are positive, a skilled person will conclude that there is a high probability of having an anti-HLA A2 antibody in the blood or plasma sample. Similarly, 4 beads (No 4, 27, 35 and 87) are grafted with an HLA A23 antigen. Consequently, if all the beads bearing an HLA A23 molecule are positive, a skilled person will conclude that there is a high probability of having an anti-HLA A23 antibody in the blood or plasma sample. Obviously, this conclusion is not definitive and has to be confirmed in Single Antigen assay (a bead only bearing HLA A2, or a bead only bearing HLA A23) assay. Therefore, pre-screening allows the selection of profiles compatible with the desired immunization, but these profiles must be confirmed by Single Antigen assay in step b).

For instance, in Example 1, HLA A23 was expressed by the transplantation donor but not by the transplantation recipient, and anti-HLA A23 antibodies were thus desired. The prescreen thus included the identification of plasma donors with high titer of anti-HLA antibodies binding to beads comprising HLA A23 antigen in a Multiple Antigens assay, for which there is a high probability that anti-HLA A23 antibodies are present.

In step a), selected plasma- or blood-donors are thus preferably those comprising anti-HLA antibodies binding to most or even all beads coated with a donor-specific HLA antigen in a Multiple Antigens assay. More preferably, plasma- or blood-donors selected in step a) are those comprising high titer anti-HLA antibodies binding to most or even all beads coated with a donor-specific HLA antigen in a Multiple Antigens assay.

When referring to anti-HLA antibodies in a plasma and/or serum sample, by "high titer in a Multiple Antigens assay" is meant an anti-HLA antibody titer in the plasma and/or serum of the donor superior to 10000 MFI, preferably superior to 12500, superior to 15000, or superior to 17500 or even preferably superior to 20000 MFI in a Multiple antigens assay, as defined above.

In all the present description, with respect to anti-HLA antibody titer, "MFI in a Multiple antigens assay" stands for mean fluorescence intensity in a Multiple Antigens assay as defined above.

When a frozen plasma, serum or blood sample of the likely donors is available, the presence of donor-specific anti-HLA antibodies may be confirmed on this sample using a Single Antigen assay, before requesting a new donation from the donor.

Alternatively, if Single Antigen assays became less expensive and plasma databases were completed by on results obtained in Single Antigen assays rather than Multiple Antigens assays, step a) above might be performed directly based on data obtained in Single Antigen assays.

In any case, the obtained results systematically need to be confirmed on new plasma, serum or blood sample in step b), since the specificities and titers of anti-HLA antibodies produced by a donor may vary with time.

In a preferred embodiment, donors are healthy females, who have developed anti-HLA antibodies following pregnancy. The large number of women immunized against HLA molecules after pregnancy offers virtually an individualized therapeutic opportunity for each patient with graft-versus-host disease, as long as there is an HLA incompatibility between donor and recipient.

In another embodiment, donors might be males or females, who have developed anti-HLA antibodies after a surgical intervention such as organ transplantation, and who have maintained an immunogenicity against such HLA-antigens even after years. This embodiment is however less preferred, since plasma is preferably taken from healthy donors rather than transplanted subjects, who might be affected by adverse effects due to plasma donation.

Preferably, step a) comprises a further step a'), in which the prescreen on databases further comprises selecting profiles without or with low titer anti-HLA antibodies directed against HLA antigens of the transplantation recipient. Indeed, it is preferable that the plasma and/or serum sample comprised in the composition for use according to the invention does not contain anti-HLA antibodies specifically binding to transplantation recipient HLA-antigen(s), since the presence of such anti-HLA antibodies specifically binding to transplantation recipient HLA-antigen(s) may result in damages against transplantation recipient's organs or cells. However, the presence of low titer anti-HLA antibodies specifically binding to transplantation recipient HLA-antigen(s) may be tolerated.

Since information available in steps a) and a') is currently based on Multiple Antigens assays, profiles without or with low titer of anti-HLA antibodies binding to beads coated with HLA antigens of the transplantation recipient in a Multiple Antigens assay are preferably selected.

When referring to anti-HLA antibodies in a plasma and/or serum sample, by "low titer anti-HLA antibodies binding to beads coated with HLA antigens of the transplantation recipient in a Multiple Antigens assay" is meant a titer in anti-HLA antibodies binding to beads coated with HLA antigens of the transplantation recipient inferior to 2000 MFI, preferably a titer inferior to 1500 MFI, inferior to 1000 MFI, inferior to 500 MFI, even preferably inferior to 300 MFI in a Multiple Antigens assay (MFI in a Multiple Antigens assay is as defined above).

For instance, in Example 1, HLA A2 was expressed by the transplantation recipient. In steps a) and a'), donors with high titer anti-HLA antibodies binding to beads bearing an HLA A23 antigen and without or low liter anti-HLA antibodies binding to beads bearing an HLA A2 antigen were thus prescreened. This further prescreen also needs to be confirmed in step b') using single antigen beads.

In second step b), a new plasma and/or serum sample of healthy donor(s) identified in step a) is screened for the presence of at least one donor-specific anti-HLA antibody. New plasma or serum samples comprising donor-specific anti-HLA antibodies are then selected in a Single Antigen assay.

If multiple plasma or serum samples comprise donor-specific anti-HLA antibodies, then plasma or serum samples comprising high titer donor-specific anti-HLA antibodies in a Single Antigen assay will be preferably selected, since this will permit to infuse the transplantation recipient with a limited volume of plasma or serum.

When referring to anti-HLA antibodies in a plasma and/or serum sample, by "high titer in a Single Antigen assay" is meant an anti-HLA antibody titer in the plasma and/or serum of the donor superior to 10000 MFI, preferably superior to 12500, superior to 15000, or superior to 17500 or even preferably superior to 20000 MFI in a Single Antigen assay, as defined above. In all the present description, with respect to anti-HLA antibody titer, "MFI in a Single Antigen assay" stands for mean fluorescence intensity in a Single Antigen assay, as defined above.

Preferably, step b) comprises a further step b'), in which the plasma and/or serum sample of healthy donor(s) identified is step a) is further screened for the absence of at least one anti-HLA antibody specifically binding to at least one transplantation recipient HLA-antigen, for reasons explained above. When step b') is performed, the plasma and/or serum sample of healthy donor(s) identified is step a) is thus selected if it does not contain or contains only low titer anti-HLA antibodies specifically binding to transplantation recipient HLA-antigen(s) in a Single Antigen assay.

When referring to anti-HLA antibodies in a plasma and/or serum sample, by "low titer in a Single Antigen assay" is meant an anti-HLA antibody titer in the plasma and/or serum of the transplantation recipient inferior to 2000 MFI, preferably a titer inferior to 1500 MFI, inferior to 1000 MFI, inferior to 500 MFI, even preferably inferior to 300 MFI in a Single Antigen assay (MFI in a Single Antigen assay is as defined above).

The plasma or serum sample of healthy donor(s) identified in step a) is screened for the presence of at least one or more (such as two, three or more, including all) donor-specific anti-HLA antibodies.

In an optional third step c), the plasma or serum sample may be diluted. This may particularly be the case when said at least one anti-HLA antibody specifically binding to at least one donor-specific HLA-antigen comprised in the plasma or serum sample is a high-titer antibody, as defined above. In this case, any appropriate pharmaceutically acceptable diluent may be used, such as saline solution.

In the context of the invention, said anti-HLA antibody derived from a blood, plasma or serum donation, may be a monoclonal or a polyclonal antibody. In most cases, it will be a polyclonal antibody.

### Compositions comprising purified anti-HLA antibodies

As an alternative to the use of appropriate plasma or serum sample, purified donor-specific anti-HLA antibodies may be used.

In one embodiment, the composition for use according to the invention may comprise at least one donor-specific anti-HLA, wherein said antibody is a monoclonal antibody.

Advantageously, said donor-specific anti-HLA monoclonal antibody may be commercially available. There exist quantities of laboratories offering for sale catalogs of antibodies, particularly antibodies directed against HLA-antigens. For example, the ATCC website that provides cell lines, commercializes some hybridomas producing anti-HLA antibodies such as the 2E12 hybridoma clone against human DR4, 2E12 (ref PTA-3798). As another example, OneLambda®, which is the leader in anti-HLA antibodies, supplies monoclonal antibodies in lyophilized form, such as reference 0544HA against HLA class I antigen A1, A11, A26+, or such as reference 0260HA against HLA class II antigen DR3, 6+. Thus, there are many options available for the person skilled in the art to find a monoclonal antibody specifically directed against a given HLA-antigen.

The use of commercial antibodies is particularly advantageous, since the large number of existing collections offers virtually an individualized therapeutic opportunity for each patient with graft-versus-host disease, as long as there is an HLA incompatibility between donor and recipient. Additionally, commercial antibodies are generally easily generated, from individual clones, stored in large amounts and immediately available. Alternatively, a skilled person may generate a collection of hybridomas or clones producing each a donor-specific anti-HLA monoclonal antibody, by technologies well-known in the art. The monoclonal donor-specific anti-HLA antibody comprised in the composition for use according to the invention may then be directly produced from an appropriate hybridoma or clone.

In any case, the donor-specific anti-HLA antibody comprised in the composition for use according to the invention is preferably a chimeric, humanized or human antibody.

In another embodiment, the composition for use according to the invention, comprises at least one donor-specific anti-HLA antibody, wherein said antibody is a polyclonal antibody.

While the composition for use according to the invention may comprise only one monoclonal or polyclonal donor-specific anti-HLA antibody, it may also comprise at least two, or three or more, donor-specific anti-HLA antibodies directed against identical or different donor-specific HLA-antigens.

### ADMINISTRATION OF THE COMPOSITION

### Doses

In the context of the invention, the composition for use in the treatment of acute Graft-versus-Host Disease (GVHD) is administered in an amount sufficient to reach a theoretical donor-specific anti-HLA antibody target titer comprised between 2500 and 10000 MFI in the blood of the transplantation recipient (MFI is as defined above).

The "theoretical target titer in the blood of the transplantation recipient" or "theoretical blood target titer" is a theoretical titer calculated depending on the titer of the donor-specific anti-HLA antibody in the composition for use according to the invention, the volume of composition administered to the transplantation recipient, and a diluting factor associated to the blood volume of the transplantation recipient.

The titer of the donor-specific anti-HLA antibody in the composition for use according to the invention is in MFI as defined above and is measured as explained above. The volume of composition administered to the transplantation recipient may vary significantly, depending on the titer of the donor-specific anti-HLA antibody in the composition for use according to the invention and the desired theoretical target titer. Depending on the volume of composition administered to the transplantation recipient, various types of intravenous administrations may be contemplated, as described below. The plasma volume of the recipient is estimated by the following formulas: Plasmatic volume = 0.065 x Weight (Kg) x (1-hematocrit) in adults or 0.08 x Weight (Kg) x (1-hematocrit) in infants and children.

Based on these values, a theoretical blood target titer is determined, which corresponds to the expected blood titer of donor-specific anti-HLA antibody in the blood of the transplantation recipient if the administered antibody freely circulated into the blood, without being adsorbed in the transplantation recipient by donor cells expressing the donor-specific HLA antigen. However, it must be understood, that when donor specific's anti-HLA antibodies are infused into the transplantation recipient, these DSA are adsorbed by circulating donor cells and notably by circulating donor T cells. In these circumstances a given DSA that has an initial titer of 10000 MFI in the donor's plasma and that is diluted 4 times upon infusion, might never reach the theoretical blood target titer of 2500 MFI. In a preferred embodiment the composition for use in the treatment of acute Graft-versus-Host Disease (GVHD) is administered in an amount sufficient to reach a titer comprised between 2500 and 5000 MFI. Indeed, this dose has surprisingly been found by the inventors to be sufficient to treat acute GVHD in a young immunocompromised child transplanted with kidney from an HLA-mismatched transplantation donor (see Example 1).

In another embodiment, the composition for use according to the invention can be administered in an amount sufficient to reach higher titers, such as titers comprised between 5000 and 7500 MFI, or between 7500 and 10000 MFI. Such target titers may notably be used in a second step, if an amount sufficient to reach a titer between 2500 and 5000 MFI is not sufficient to efficiently treat acute GVHD.

### Routes and schemes of administration

The composition for use according to the invention is preferably administered intravenously.

The person skilled in the art knows that there are two ways to increase the final theoretical target titer of donor-specific anti-HLA antibody(ies) in the transplantation recipient, depending on the titer of the donor-specific anti-HLA antibody(ies) in the composition and on the desired theoretical target titer in the transplantation recipient's blood.

Notably, the first way is to use a composition (e.g. a plasma or a serum, or a monoclonal antibody) with a higher initial titer (in MFI as defined above) in the donor-specific anti-HLA antibody(ies). In this case, a small to moderate volume (depending on the targeted titer in transplantation recipient's blood) of the composition for use according to the invention may be directly administered to the patient, without any expected adverse effect.

However, when the composition for use according to the invention comprises only a moderate or low titer (in MFI as defined above) donor-specific anti-HLA antibody(ies), larger volume of composition is needed to reach the targeted titer in transplantation recipient's blood. This situation will mainly occur when there is no high titer plasma or serum that can be used for the implementation of the composition according to the invention. However, excessive volume (> 15-20 ml/kg) cannot be infused as it may create plasma mass inflation that is too high for the heart; in this case, in particular when plasma samples are used, plasma exchanges involving substituting the transplantation recipient's plasma with that of the plasma- or blood-donor should be used. This second technique is particularly advantageous as it allows to reach a target antibody titer in the transplantation recipient close to that of the undiluted plasma sample.

Thus, in a particular embodiment, the composition for use in the treatment of acute Graft-versus-Host Disease (GVHD) is infused directly into the blood flow of the transplantation recipient. This mode of administration is particularly advantageous when the titer of donor-specific anti-HLA antibodies in the composition for use according to the invention (which may comprise plasma, serum, monoclonal or polyclonal antibody) to be administered is high, such as superior to 10000 MFI, preferably superior to 12500, superior to 15000, or superior to 17500 or even preferably superior to 20000 MFI.

This mode of administration is also particularly advantageous when the target titer of donor-specific anti-HLA antibodies to reach in the transplantation recipient's blood is a moderate titer.

By "moderate titer" is meant an anti-HLA antibody titer in blood of the transplantation recipient comprised between 2500 and 10000 MFI, such as 7500, 5000, 4000, 3000 MFI, or such as 2500 MFI.

In another particular embodiment, when the composition for use in the treatment of acute Graft-versus-Host Disease (GVHD) comprises plasma, it may be administered via plasma exchanges. This mode of administration is particularly advantageous when the titer of specific anti-HLA antibodies in the plasma to be administered is low, or when the target titer of donor-specific anti-HLA antibodies to reach in the transplantation recipient's plasma is high.

According to the invention, the composition for use in the treatment of acute Graft-versus-Host Disease (GVHD) may be administered one or several times to the subject. In a particular embodiment, said composition for use in the treatment of acute Graft-versus-Host Disease (GVHD) is administered only one time to the transplantation recipient. Accordingly, said composition is administered in an amount sufficient to reach a titer comprised between 2500 and 5000 MFI, or between 5000 and 7500 or between 7500 and 10000 MFI.

In another particular embodiment, said composition for use in the treatment of acute Graft-versus-Host Disease (GVHD) is administered several times to the transplantation recipient, such as two times, three times, four times or even more. Preferably, the use of several administrations may intend to gradually increase the titer in donor-specific anti-HLA antibodies in the transplantation recipient, until the titer is sufficient to treat acute GVHD. In this case, said composition can be administered gradually such that after the second administration the titer is higher than after the first administration, and each supplemental administration allows to increase or to maintain the titer of said anti-HLA antibody in the blood of the transplantation recipient.

Alternatively, the use of several administrations may intend to maintain a target titer of donor-specific anti-HLA antibodies in the transplantation recipient during an extended time period. In this case, said composition is administered in an amount sufficient to reach and to maintain a titer comprised between 2500 and 5000 MFI, or between 5000 and 7500 or between 7500 and 10000 MFI.

However, when testing the administration of donor-specific anti-HLA antibodies for the treatment of acute GVHD, the inventors found that transient presence of donor-specific anti-HLA antibodies was sufficient to durably abate graft versus host (GvH) reactivity and treat acute GVHD, presumably by targeting activated donor T cells. As a result, when several administrations of the composition for use according to the invention are made, they are preferably interspaced by a few hours (such as at least 6 hours) to a few weeks (such as at most 4 weeks), and more preferably by a few hours (such as at least 24 hours) to a few days (such as at most 10, 9, 8, 7, 6, 5, 4, or 3 days). Preferably, they are also all performed within a period that does not exceed a few weeks (such as at most 4 weeks), more preferably within a period that does not exceed a few days (such as at most 10, 9, 8, 7, 6, 5, 4, or 3 days).

### USE AFTER SOLID ORGAN TRANSPLANTATION

### General use after solid organ transplantation

In solid organ transplantation, acute GVHD is rare for three reasons: (1) the number of immune cells of the donor, present in the graft and able to migrate from the graft to the host, is limited, (2) both the donor and recipient immune cells are immunosuppressed and (3) the recipient's lymphocyte count is considered much higher than that of the donor; thus, if an insufficient immune-suppressor treatment allows an allo-immune response to develop, the recipient's immune cells should easily take over those of the donor.

As a consequence, in organ transplantation, acute GVHD is mainly described after intestinal transplantation in 5 to 10% of patients. The risk is increased in the case of a multivisceral transplant (small bowel + liver + pancreas), especially if the spleen, which is an important provider of immune cells, is included. Mortality is however greater than 50%, especially when recipients are immunocompromised. Although rare, acute GVHD after solid organ transplantation is thus a life-threatening condition with unsatisfying treatment options.

In the context of the invention, the composition may thus be for use after allogenic transplantation with at least one solid organ from an HLA-mismatched transplantation donor, wherein said at least one solid organ is preferably selected from kidney, small bowel, liver, pancreas, spleen, lung and their combinations.

In a particular embodiment, the composition may be for use after allogenic transplantation with kidney. As indicated above, in case of solid organ transplantation, acute GVHD is mainly described after intestinal transplantation, and the risk of acute GVHD is increased in case of multivisceral transplant (small bowel + liver + pancreas), especially if the spleen, which is an important provider of immune cells, is included. Therefore, the composition may also be for use after allogenic transplantation with small bowel, or after allogenic multivisceral transplantation with small bowel and at least one other solid organ selected from liver, pancreas, and spleen.

In the present invention, in the context of allogenic transplantation with at least one solid organ, said at least one donor-specific anti-HLA antibody is a donor-specific anti-HLA class I or a donor-specific anti-HLA class II antibody.

Indeed, the use of the composition aims to target and to remove activated lymphocytes from the organ donor, and such activated lymphocytes carry on their surface both class I and class II antigens. In addition, while donor organ cells express HLA class I molecules and may thus also be targeted by donor-specific anti-HLA antibodies, only a high and chronic titer of antibodies to donor organ cells may generally lead to chronic organ rejection. Since only transient presence of moderate titers of donor-specific anti-HLA antibodies was found by the inventors to be sufficient to permanently remove donor activated T cells and treat acute GVHD, donor-specific anti-HLA class I antibodies may be used in the context of solid organ transplantation. This is confirmed in Example 1. However, since activated T lymphocytes that should be removed by donor-specific anti-HLA antibodies also express HLA class II antigens, contrary to most other donor organ cells, donor-specific anti-HLA class II antibodies may preferably be used.

### Patients with severe primary immunodeficiency

According to one embodiment of the invention the composition may be for use in the treatment of acute Graft-versus-Host Disease (GVHD) in a transplantation recipient after allogenic solid organ transplantation, wherein said transplantation recipient suffers from severe primary immunodeficiency, i.e. involving T lymphocytes (functional impairment and/or quantitative deficiency), such as SCID, DiGeorge Syndrome, Ataxia-Telangiectasia.... T cell deficiency may result from genetic disorders in cytokine signaling, T-cell receptor signaling, co-stimulation, immunometabolism, and thymic education.

"SCID" or "severe combined immune deficiency" is a collective name for the most severe forms of congenital immune deficiency and is characterized by an immune system that functions very poorly or not at all due to combined absence of T-lymphocyte and B-lymphocyte function as well as in many cases absence of NK lymphocytes function too.

Among the different characterized forms of SCID, the most prevalent is the X-linked severe combined immunodeficiency. Other forms include adenosine deaminase deficiency, purine nucleoside phosphorylase deficiency, bare lymphocyte syndrome, reticular dysgenesis, Omenn syndrome (RAG-1 and RAG-2 gene deficiency) and others. Additionally, many other cases have an etiology, a genetic cause, still unknown.

SCID patients are usually affected by severe bacterial, viral, or fungal infections early in life and often associated with interstitial lung disease, chronic diarrhea, and failure to thrive. Patients suffering from this syndrome are forced to live in a sterile environment, sometimes for their entire lives, as they are extremely vulnerable to infections.

The transplantation of hematopoietic stem cells is the only effective curative treatment to severe combined immune deficiency.

Primary immune deficiency is an important factor to consider during solid organ transplantation. In this case, transplantation recipient has a very limited number of lymphocytes, which can easily be outnumbered by lymphocytes coming from the grafted tissues, and thus may not able to eradicate even very low numbers of donor lymphocytes. The present invention is thus particularly applicable in this context.

### USE AFTER HEMATOPOIETIC CELLS TRANSPLANTATION

When transplanting non-identical HLA hematopoietic cells (HC), large numbers of immune cells from the donor are transferred into the recipient and the risk of acute GVHD is major, occurring in 20 to 50% of cases. Mortality remains very high, greater than 30%, linked to major infectious risks. Notably, infections result from the breakdown of intestinal mucosal barriers and the strengthening of immunosuppressive treatment. There was thus a need for alternative treatment of acute GVHD after allogenic transplantation with hematopoietic cells from an HLA-mismatched transplantation donor. This need is fulfilled by the composition for use according to the invention, which permit treating life-threatening acute GVHD by a minimally invasive treatment.

In the context of the invention, the composition may thus be for use after allogenic transplantation with hematopoietic cells from an HLA-mismatched transplantation donor, preferably after allogenic bone marrow transplantation or allogenic hematopoietic stem cell (HSC) transplantation from an HLA-mismatched transplantation donor or blood transfusion from an HLA-mismatched transplantation donor.

By "bone marrow" (abbreviated as "BM") is meant the semi-solid tissue which may be found within the spongy or cancellous portions of bones. In adult humans, bone marrow is primarily located in the ribs, vertebrae, sternum, and bones of the pelvis. It is composed of hematopoietic cells, marrow adipose tissue, and supportive stromal cells. Bone marrow is the primary site of hematopoiesis leading to both myeloid and lymphoid lineages. Bone marrow transplantation is often used as a treatment for hematological malignancies, bone marrow failure (aplastic anemia), severe primary immunodeficiency, hemoglobinopathies (sickle cell disease, thalassemia), and inherited metabolic disorders. The recipient is then first irradiated to eliminate all his/her immune cells, and then transplanted with donor bone marrow. In case of HLA-mismatched bone marrow transplantation, the amount of immunocompetent lymphoid cells of the recipient is thus null or very low, while a huge number of immunocompetent lymphoid cells of the donor is administered, thus explaining the high occurrence of acute GVHD.

By "hematopoietic stem cells" or "HSCs" is meant immature hematopoietic cells that may give rise to both the myeloid and lymphoid lineages of blood cells. Hematopoietic stem cells are mainly found in the bone marrow and the peripheral blood. Due to their property to regenerate all white blood cells, they may be used instead of bone marrow for the treatment of hematological malignancies, bone marrow failure (aplastic anemia), severe primary immunodeficiency, hemoglobinopathies (sickle cell disease, thalassemia), and inherited metabolic disorders.

Blood contains hematopoietic cells, and blood transfusion is thus herein considered as a transplantation of hematopoietic cells. By "blood transfusion" is meant the injection of blood by intravenous infusion.

In the present invention, in the context of allogenic transplantation with hematopoietic cells from an HLA-mismatched transplantation donor, said at least one anti-HLA antibody is a donor specific anti HLA class I or class II antibody, and more preferably a donor-specific anti-HLA class II antibody.

Indeed, in bone marrow or hematopoietic stem cells (HSCs) (bone marrow HSCs or peripheral HSCs) transplantation, it is important to preserve the donor's HSCs, as well as neutrophils, platelets, and lymphocytes which are non-reactive against the recipient. These cells constitute a population of isolated cells all carrying class I HLA-antigens and therefore sensible to anti-HLA class I antibodies.

However, once the HC (in particular BM or HSC) is engrafted, which can be monitored by the reconstitution of neutrophils and monocytes populations in the recipient, class I anti-HLA antibodies may no longer be a risk for the HC (in particular BM or HSC) transplant.

For these reasons, donor-specific anti-HLA class II antibodies are preferred, but both anti-HLA class I and anti-HLA class II antibodies can be used if GVHD occurs after HC (in particular BM or HSC) engraftment, in particular after reconstitution of neutrophils and monocytes populations by donor hematopoietic cells. When GVHD occurs before HC (in particular BM or HSC) engraftment, donor-specific anti-HLA class II antibodies will preferably be used.

In the context of blood transfusion, a severe primary immunodeficiency is also a risk factor for acute GVHD. In this case, transplantation recipient has a very limited number of lymphocytes, which can easily be outnumbered by lymphocytes coming from the transfused blood, and thus may not able to eradicate even very low numbers of donor lymphocytes. The present invention is thus particularly applicable in this context.

### CORTICOSTEROID RESISTANCE OR DEPENDENCE

According to an embodiment of the invention, the composition is for use in the treatment of acute Graft-versus-Host Disease (GVHD) in a transplantation recipient after allogenic transplantation, wherein said acute GVHD is a corticosteroid resistant GVHD or a corticosteroid dependent GVHD.

Indeed, as explained before, treatment of acute GVHD is currently based primarily on the administration of high doses of corticosteroids, sometimes in combination with calcineurin inhibitors. However, a number of patients are corticosteroid-resistant or become corticosteroid dependent. For these patients, high morbidity-mortality may be expected, and an alternative treatment is particularly needed. The composition for use according to the invention fulfils this need.

The following examples merely intend to illustrate the present invention.

### EXAMPLES

### EXAMPLE 1. ACUTE GVHD AFTER KIDNEY TRANSPLANTATION IN A 3.5-YEAR-OLD CHILD

We were confronted with a very severe corticosteroid-resistant graft-versus-host disease after kidney transplantation in a 3.5-year-old child with severe primary immune deficiency. The progressive nature of hepatic (icteric cholestasis) and hematological (pancytopenia) disorders, under high doses of steroids and tacrolimus, the lack of therapeutic resources (and hematological toxicity of ruxolitinib) and the deterioration of the general and nutritional state have led us to propose an innovative therapy, the only alternative to a palliative approach. This treatment allowed not only a very rapid amendment of life-threatening conditions, but also a decrease in immunosuppressive treatment.

### Materials and Methods

### Patient

The patient was a 3.5-year-old boy, diagnosed with Schimke immune-osseous dysplasia, a rare autosomal recessive DNA repair disorder caused by SMARCAL1 gene mutation. Clinical features include short stature, spondyloepiphyseal dysplasia, steroid-resistant nephrotic syndrome, cerebral ischemic strokes and T-cell deficiency. He received a first kidney transplant from a 25-year-old male deceased donor. The option of prior or combined bone marrow transplantation had been previously discussed yet rejected because of the reportedly high mortality in this disease. His immunosuppressive regimen included basiliximab induction therapy, and a combination of tacrolimus, azathioprine and steroids. In the context of primary immune deficiency, the persistence of a profound lymphopenia led to azathioprine and prednisone withdrawal at Post-Operative Day (POD) 20 and 31, respectively and eventually to tacrolimus discontinuation on POD 81. Within the subsequent weeks, he progressively developed a febrile full-blown acute grade IV GVHD, targeting the skin, gut, liver and bone marrow. High donor chimerism (>99%) was found in circulating T cells. Steroid and tacrolimus therapies were resumed on POD 94 and POD 104, respectively. High doses of steroid cleared skin lesions, fever and improved diarrheal output. However, 4 weeks later, persisting intestinal impairment still precluded enteral feeding, entailing a degradation of nutritional status, associated with apathetic attitude and breakdown of the circadian sleep-wake cycle. In the meantime, cholestatic jaundice (total bilirubin 12.1 mg/dL) and hematological abnormalities (platelets 13 G/L, white blood cells 500 /mm3) had been steadily worsening. The liver biopsy confirmed hallmark features of active GVHD, including bile duct injury, intra-epithelial lymphocytes and sparse lymphocytic infiltrates. Ruxolitinib or T-cell depleting agents were considered at too high infectious risk in this setting of primary immune deficiency.

### Donor chimerism assessment

Monoclonal HLA class I allele-specific antibodies were used to discriminate HLA-A23+ donor-specific (clone BIH0964) and HLA-A2+ recipient-specific (clone FH0037, One Lambda, Canoga Park, CA) cells, in combination with a pan-HLA ABC (class I) antibody, as previously reported (5-6). Donor (KMR045) and recipient (KMR047)-specific single nucleotide polymorphisms were also quantified in blood and bone marrow cells using real-time quantitative polymerase chain reaction (KMRtype® and KMRtrack® chimerism monitoring reagents, Gendx). Recipient sera and donor plasma samples were tested using the screening and the class I and class II Single Antigen flow bead assays for anti-HLA antibodies (One Lambda®, Canoga Park, CA), according to the manufacturer's instructions.

### Nationwide plasma screening

We inquired the French National Blood Service (EFS) on POD115 about a possible directed donation of DSA-rich plasma from a healthy individual. Once authorizations had been granted by the French National Agency for Medicines and Health Product Safety (ANSM) and by an ad hoc Ethical Committee, the EFS launched a nationwide call within the 16 EFS-endorsed HLA laboratories (Figure 1A). At the end of the selection process, DSA-rich plasmas from two different donors were allocated to our institution (Figure 1B). The first plasma (#1) contained a high-level DSA targeting the 82LR epitope, shared by all Bw4-associated HLA class I molecules including the donor HLA-A23 antigen, but none of the recipient's ones (Figure 1B). The second plasma (#2) included DSA recognizing HLA-A23, A24 and a few other Bw4-associated molecules (Figure 1B). Plasma #1 also contained an irrelevant non-donor-reactive anti-DQ6 antibody that could be used as internal control post-infusion.

### Plasma infusions

Plasma was administered after obtaining a written-informed consent from the parents. Given the potential of high-level DSA to harm the kidney allograft, we opted for a stepwise protocol based on a gradual increase in the DSA target level, as assessed by single-antigen assay Mean Fluorescence Intensity (MFI). The first step aimed at targeting an in vivo MFI value comprised between 2,500 and 5,000. Patient's plasma volume being estimated at around 600 mL, a plasma infusion of 200 mL (20mL/kg) would dilute 4 times the HLA-A23 DSA. The DSA was measured at 4439 and 3907 MFI units in the 4-fold diluted plasmas #1 and #2, respectively (Figure 1C). Although the antibodies, present at high level (MFI>3000) in therapeutic plasmas (Figure 1B), peaked in patient's serum 2 hours following infusion, the others displayed decreased levels through dilution (Figures 1D). Two hours after plasma #1 infusion, the irrelevant anti-DQ6 antibody achieved a level roughly similar to that obtained by an ex vivo 4-fold plasma dilution, whereas anti-HLA-A23 DSA, presumably adsorbed on donor cells, reached much lower levels (Figure 1C). Rapid adsorption on donor cells was also supported by the further drop in DSA MFI at 24 hours post-infusion (Figure D).

### Results

### Clinical response to donor-targeted serotherapy

The patient received 200 mL of plasma #1 and plasma#2 on POD126 and POD129 (Day0 and day3 in Figure 2), respectively. The two plasma infusions were remarkably well tolerated, especially with regards to kidney allograft. The patient had been experiencing severe neutropenia (<0.5 G/L), despite intensive G-CSF support, and major hyperbilirubinemia (>10 mg/dL) for 15 and 6 days, respectively (Figure 2A). The day following the infusion, white cell count rose sharply, meanwhile the bilirubin dropped. At day6 post-infusion, white cell counts peaked at 5.9 G/L, while total bilirubin decreased to 3.8 mg/dL (Figure 2A). The last red blood cell pack was administered on POD138 and the patient remained transfusion-independent thereafter thanks to a reticulocyte burst (Figure 2A). In a very short lapse of time, the general status dramatically improved. Diarrhea completely and durably resolved, accompanied with albuminemia normalization (Figure 2A), and the child was able to gain 2 kg over 5 weeks. Steroid doses were progressively tapered down (Figure 2A). At 0.5 mg/kg, an augmentation of □GT levels required a transient increase in steroid before further reduction to 0.15 mg/kg (on POD290).

### Immunological response to donor-targeted serotherapy

Before the first plasma infusion, roughly 99% of the circulating CD3+ T cells were donor-derived. An unusual staining pattern was noticed in a subset of donor T cells that co-expressed the recipient-specific HLA-A2 molecule (subset II), yet at a lower level compared with recipient T cells (subset I, Figure 2B). This T cell subset displayed the activation markers CD69, CD25 and HLA-DR (not shown). Imaging of these cells (Amnis® ImageStream) unveiled that this pattern resulted from recipient-derived extracellular microvesicles (23, 25) bound to donor T cells (Figure 2C). Some recipient microvesicles co-stained with CD14 (monocyte-derived), but most of them remained of uncharacterized origin (Figure 2D). Strikingly, this T cell subset sharply decreased as early as 3 days after the first infusion and was barely sizeable thereafter (Figure 2B).

### Hematopoietic reconstitution from donor-derived progenitors

After a complete remission of GVHD had been obtained, donor chimerism remained unexpectedly high, over 99% and 96% in total blood and T cells (Figure 3A), respectively. Similarly, the red blood type changed to the donor type between POD131 and POD219, as evidenced by the switch from the C+ to C- Rhesus antigen (Figure 3B), while a full and durable donor chimerism was observed in all lymphoid and myeloid lineages (Figure 3C). Taken together, these findings strongly suggested that donor-derived hematopoietic stem and progenitor cells (HSPC) were able to support hematopoiesis. Consistent with this, donor chimerism was measured in magnetically-sorted CD34+ HSPC bone marrow cells at roughly 96% with both quantitative PCR and flow cytometry on POD156. However, thorough flow-cytometry analysis, according to the current model of bone marrow lineage determination, showed that donor chimerism greatly varied along the hematopoietic differentiation pathway (Figure 3C). Although host-derived cells still accounted for 72% of the most immature hematopoietic stem cells (HSC) their contribution dropped to 42% in the downstream multipotent progenitors (MPP) and far less among more committed progenitors (Figure 3C).

### Conclusions

All these findings suggest that this passive allogeneic immunotherapy approach may provide an innovative alternative for the treatment of acute graft-versus-host disease after transplantation of non-identical HLA hematopoietic organ or cells (such as hematopoietic cells). The transfer of -donor-specific anti-HLA antibodies preferentially targeted the donor's activated T cells and preserved immune reconstitution from donor hematopoietic precursors. Neutrophils (of the donor's phenotype) are released within 24 hours after the first plasma infusion. In this respect, it is important to note that the expression density of HLA class I by neutrophils is ten times lower than that of activated lymphocytes and monocytes, involved in graft-versus-host disease.

This observation provides proof of concept on the value of passive donor immunotherapy in the event of graft disease against the severe cortico-resistant or cortico-dependent host during organ or hematopoietic cell transplantation. It could become a reference therapeutic strategy for acute graft-versus-host diseases occurring after organ transplantation, particularly intestinal and multi-visceral diseases. In addition, the differential effect of DSA on graft-versus-host response and immune reconstitution from donor hematopoietic precursor cells suggests that this strategy is transposable to acute graft-versus-host diseases occurring after transplantation of identical non-HLA allogeneic hematopoietic cells. Furthermore, in this scenario, the therapeutic strategy would only require minor adaptations such as the use of donor-specific class II HLA-antibody(ies).

### BIBLIOGRAPHIC REFERENCES

1. Almagro et al. Frontiers in Bioscience 13, 1619-1633, January 1, 2008.
2. Chen XB, Yang J, Xu MQ, Wen TF, Yan LN. Unsuccessful treatment of four patients with acute graft-vs-host disease after liver transplantation. World J Gastroenterol. 2012 Jan 7;18(1):84-9. doi: 10.3748/wjg.v18.i1.84.
3. Bruggermann et al., Year in Immuno., 7:33 (1993).
4. Duchosal et al. Nature 355:258 (1992).
5. Fischer RT, Friend B, Talmon GA, Grant WJ, Quiros-Tejeira RE, Langnas AN, Coccia PF, Intestinal transplantation in children with multiple intestinal atresias and immunodeficiency. Pediatric Transplantation, 18, 2, (190-196), (2014).
6. Hoogenboom et al., J. Mol. Biol., 227:381 (1991).
7. Jakobovits et al., Nature, 362:255-258 (1993) (b).
8. Jakobovits et al., Proc. Natl. Acad. Sci. USA. 90:2551 (1993) (a).
9. Marks et al., J. Mol. Biol., 222:581- 5 597 (1991).
10. Mazariegos GV, Abu-Elmagd K et al. Graft versus host disease in intestinal transplantation. Am J Transplant 2004 (doi: 10.1111/j.1600-6143.2004.00524.x)
11. Patel R, Terasaki PI. Significance of the positive crossmatch test in kidney transplantation. N Engl J Med. 1969 Apr 3;280(14):735-9.
12. Perri R, Assi M, Talwalkar J, Heimbach J, Hogan W, Moore SB, Rosen CB. Graft vs. host disease after liver transplantation: a new approach is needed. Liver Transpl. 2007 Aug;13(8):1092-9.
13. US 5,591,669,
14. US 5,598,369,
15. US 5,545,806,
16. US 5,545,807,
17. US 6,150,584
18. Vaughan et al. Nature Biotech 14:309 (1996).
19. Verhoeyen et al. BioEssays, 8:74, 1988.
20. Verhoeyen et al. Science, 239:1534-1536, 1988.
21. Socié G, et al., A phase 3 randomized trial comparing inolimomab vs usual care in steroid-resistant acute GVHD. Blood. 2017 Feb 2;129(5):643-649. doi: 10.1182/blood-2016-09-738625. Epub 2016 Nov 29.
22. Triulzi DJ et al., The effect of previous pregnancy and transfusion on HLA alloimmunization in blood donors: implications for a transfusion-related acute lung injury risk reduction strategy, Transfusion 49(9): 1825-35, 2009.
23. Zuber J, Rosen S, Shonts B et al. Macrochimerism in intestinal transplantation association with lower rejection rates and multivisceral transplant without GVHD. Am J Transplant 2015 (doi: 10.1111/ajt. 13325.)
24. Zuber J, Shonts B, Lau SP et al. Bidirectional intragraft alloreactivity drives the repopulation of human intestinal allografts and correlates with clinical outcome. Sci Immunol 2016 (doi: 10.1126/sciimmunol.aah3732.)

## Claims

1. A composition for use in the treatment of acute Graft-versus-Host Disease (GVHD) in a transplantation recipient after allogenic transplantation with at least one solid organ and/or hematopoietic cells (HC) from a human leucocyte antigen (HLA)-mismatched transplantation donor, wherein said composition comprises at least one donor-specific anti-HLA antibody.

2. The composition for use according to claim 1, wherein said composition comprises plasma or serum from a sensitized plasma-donor or blood-donor producing donor-specific anti-HLA antibodies, preferably said sensitized plasma-donor or blood-donor is a healthy female plasma-donor or blood-donor, whose blood, plasma or serum has previously been screened for anti-HLA antibodies following pregnancy.

3. The composition for use according to claim 2, wherein said sensitized plasma-donor or blood-donor has a compatible blood group with respect to the transplantation recipient.

4. The composition for use according to claim 2 or claim 3, wherein said composition has been obtained beforehand by:
a) interrogating databases including healthy female plasma or blood donors, whose blood, plasma or serum had been screened for anti-HLA antibodies following pregnancy, and selecting at least one healthy female plasma-donor or blood-donor likely to produce a donor-specific anti-HLA antibody,
b) testing plasma or serum sample(s) from the selected healthy female plasma-donor(s) or blood-donor(s) for anti-HLA antibodies and selecting the plasma or serum sample if said plasma or serum sample comprises donor-specific anti-HLA, and
c) optionally, diluting the plasma or serum sample.

5. The composition for use according to claims 2 to 4, wherein said composition is administered by plasma infusion or by plasma exchanges.

6. The composition for use according to claim 1, wherein said antibody is a monoclonal antibody.

7. The composition for use according to any one of claims 1 to 6, wherein the composition is administered in an amount sufficient to reach a theoretical donor-specific anti-HLA antibody target titer comprised between 2500 and 10000 MFI, such as 2500 to 5000 MFI, 5000 to 7500 MFI or 7500 to 10000 MFI, in the blood of the transplantation recipient.

8. The composition for use according to any one of claims 1 to 7, wherein the composition is administered one or several times to the transplantation recipient.

9. The composition for use according to any one of claims 1 to 8, after allogenic transplantation with at least one solid organ from an HLA-mismatched transplantation donor, wherein said at least one solid organ is preferably selected from kidney, small bowel, liver, pancreas, spleen, lung and their combinations.

10. The composition for use according to claim 9, after allogenic transplantation with kidney or small bowel, or after allogenic multivisceral transplantation with small bowel and at least one other solid organ selected from liver, pancreas, and spleen.

11. The composition for use according to claim 9 or 10, wherein said at least one donor-specific anti-HLA antibody is a donor-specific class I or a class II donor-specific anti-HLA antibody.

12. The composition for use according to any one of claims 9 to 11, wherein said transplantation recipient suffers from primary immunodeficiency, in particular a primary immunodeficiency of T lymphocytes, such as SCID.

13. The composition for use according to any one of claims 1 to 8, after allogenic transplantation with hematopoietic cells from an HLA-mismatched transplantation donor, preferably after allogenic bone marrow transplantation, allogenic hematopoietic stem cell (HSC) transplantation from an HLA-mismatched transplantation donor, or blood transfusion from an HLA-mismatched transplantation donor.

14. The composition for use according to claim 13, wherein said at least one donor-specific HLA-antigen specifically bound by the antibody is a class II donor-specific HLA-antigen

15. The composition for use according to any one of claims 1 to 14, wherein said acute GVHD is a corticosteroid resistant GVHD or a corticosteroid dependent GVHD.
